(19) Europäisches Patentamt
European Patent Office
Office européen des brevets

(11) EP 1 174 421 A1

(12) EUROPEAN PATENT APPLICATION
published in accordance with Art. 158(3) EPC

(43) Date of publication:
23.01.2002 Bulletin 2002/04

(21) Application number: 00911405.9

(22) Date of filing: 28.03.2000

(51) Int Cl.7: **C07C 311/46**, C07C 311/19, C07C 311/29, C07D 207/12, C07D 211/26, A61K 31/40, A61P 7/02, A61P 43/00, A61K 31/445, A61K 31/18

(86) International application number:
PCT/JP00/01891

(87) International publication number:
WO 00/59876 (12.10.2000 Gazette 2000/41)

(84) Designated Contracting States:
AT BE CH CY DE DK ES FI FR GB GR IE IT LI LU MC NL PT SE
Designated Extension States:
AL LT LV MK RO SI

(30) Priority: 31.03.1999 JP 9395199

(71) Applicant: Kissei Pharmaceutical Co., Ltd.
Matsumoto-shi Nagano 399-8710 (JP)

(72) Inventors:
- Akahane, Satoshi
  Matsumoto-shi, Nagano 399-0033 (JP)
- Uchida, Masahiko
  Minamiazumi-gun, Nagano 399-8302 (JP)
- Isawa, Hidetoshi
  Sakai-gun, Fukui 910-4104 (JP)
- Kikuchi, Norihiko
  Matsumoto-shi, Nagano 390-1242 (JP)
- Kai, Yuichiro
  Minamiazumi-gun, Nagano 399-8303 (JP)
- Hara, Kiyoto
  Edogawa-ku, Tokyo 134-0081 (JP)
- Honma, Toshiki, Windia Hakuya 203
  Minamiazumi-gun, Nagano 399-8304 (JP)

(74) Representative:
Baverstock, Michael George Douglas et al
BOULT WADE TENNANT, Verulam Gardens 70
Gray's Inn Road
London WC1X 8BT (GB)

# (54) 3-AMIDINOBENZENESULFONAMIDE DERIVATIVES, MEDICINAL COMPOSITIONS CONTAINING THE SAME AND INTERMEDIATES IN THE PRODUCTION THEREOF

(57) The present invention relates to 3-amidinobenzenesulfonamide derivatives represented by the general formula:

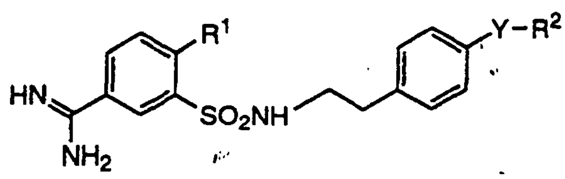

wherein $R^1$ represents a hydrogen atom or a hydroxy group; Y represents a single bond or an oxygen atom; and $R^2$ represents a lower alkyl group or a group represented by the general formula:

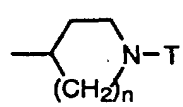

(wherein n represents 1 or 2; and T represents a hydrogen atom, a lower acyl group, a halo (lower acyl) group or a

EP 1 174 421 A1

group represented by the general formula:

-C(=NH)-W

(wherein W represents a lower alkyl group)), and pharmaceutically acceptable salts thereof, which have an excellent activated blood coagulation factor X inhibitory activity and are useful as activated blood coagulation factor X inhibitors, and to pharmaceutical compositions comprising the same and intermediates thereof.

## Description

### Technical Field

The present invention relates to novel 3-amidinobenzenesulfonamide derivatives and pharmaceutically acceptable salts thereof which are useful as medicaments, pharmaceutical compositions comprising the same and their intermediates.

### Background Art

Anticoagulation therapy has been extensively performed for the prevention and treatment of thromboembolic diseases caused by accelerating blood clotting, and drugs such as heparin and warfarin potassium are frequently used as anticoagulant agents at present.
However, it is known that heparin is apt to cause bleeding tendency since it has antithrombin and activated blood coagulation factor X inhibitory activities.
Warfarin potassium is an anticoagulant which controls biosynthesis of vitamin K-dependent coagulation factor, and it is difficult to control the anticoagulation capacity due to its action mechanism when this drug is used in the prevention and treatment of thromboembolic diseases. Therefore, this drug is extremely difficult to use clinically.
In recent years, selective thrombin inhibitors have been developed and have been used clinically. However, since thrombin plays a close part in the conversion of fibrinogen into fibrin in blood coagulation cascade reactions and platelet activation and aggregation, thrombin inhibitors also have similar problems to those of heparin from the safety point of view, such as bleeding tendency and it has been reported that their effects are not necessarily enough.
On the other hand, activated blood coagulation factor X, which acts at the joining point of the extrinsic and intrinsic blood coagulation cascade reactions, locates upstream to thrombin, so that the coagulation inhibition is more efficient than that of thrombin inhibitors and therefore activated blood coagulation factor X inhibitors attract public attention as drugs having a possibility of inihibiting the coagulation system effectively.
Furthermore, with the changes in European and American life styles and the increase in aged population in recent years, incidence of thromboembolic diseases such as myocardial infarction and arteriovenous obstruction will go on increasing, and therefore, demands on development of more effective anticoagulants are great and social importance of their treatment has been increasing more and more.

### Disclosure of Invention

The present inventors have extensively studied in order to find novel compounds having an excellent activated blood coagulation factor X inhibitory activity. As a result, it has been found unexpectedly that certain 3-amidinobenzenesulfonamide derivatives have a potent and selective activated blood coagulation factor X inhibitory activity, thereby forming the basis of the present invention.
Accordingly, the present invention relates to a 3-amidinobenzenesulfonamide derivative represented by the general formula:

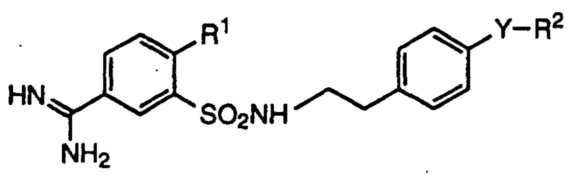
(I)

wherein $R^1$ represents a hydrogen atom or a hydroxy group; Y represents a single bond or an oxygen atom; and $R^2$ represents a lower alkyl group or a group represented by the general formula:

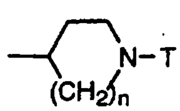

(wherein n represents 1 or 2; and T represents a hydrogen atom, a lower acyl group, a halo (lower acyl) group or a group represented by the general formula:

-C(=NH)-W

(wherein W represents a lower alkyl group)), or a pharmaceutically acceptable salt thereof.

The present invention relates to a pharmaceutical composition comprising a 3-amidinobenzenesulfonamide derivative represented by the above general formula (I) or a pharmaceutically acceptable salt thereof.

The present invention relates to an activated blood coagulation factor X inhibitor which comprises, as an active ingredient, a 3-amidinobenzenesulfonamide derivative represented by the above general formula (I) or a pharmaceutically acceptable salt thereof.

The present invention relates to a method for the prevention or treatment of thromboembolic diseases which comprises administering a 3-amidinobenzenesulfonamide derivative represented by the above general formula (I) or a pharmaceutically acceptable salt thereof.

The present invention relates to a use of a 3-amidinobenzenesulfonamide derivative represented by the above general formula (I) or a pharmaceutically acceptable salt thereof for the manufacture of a pharmaceutical composition for the prevention or treatment of thromboembolic diseases.

The present invention relates to a process for the manufacture of a pharmaceutical composition for the prevention or treatment of thromboembolic diseases, characterized in the use, as an essential constituent of said pharmaceutical composition, of a 3-amidinobenzenesulfonamide derivative represented by the above general formula (I) or a pharmaceutically acceptable salt thereof.

The present invention relates to a 3-cyanobenzene-sulfonamide derivative represented by the general formula:

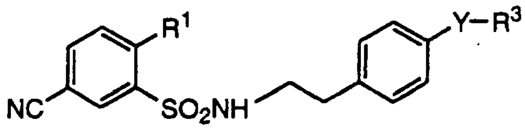

(II)

wherein $R^1$ represents a hydrogen atom or a hydroxy group; Y represents a single bond or an oxygen atom; and $R^3$ represents a lower alkyl group or a group represented by the general formula:

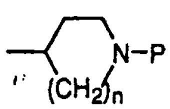

(wherein n represents 1 or 2; and P represents a hydrogen atom, a lower acyl group, a halo (lower acyl) group, a group represented by the general formula:

-C(=NH)-W

(wherein W represents a lower alkyl group) or an amino-protective group), or a salt thereof.

In the present invention, the term "lower alkyl group" means a straight-chained or branched alkyl group having 1 to 6 carbon atoms such as a methyl group, an ethyl group, a propyl group, an isopropyl group, a butyl group, an isobutyl group, a sec-butyl group, a pentyl group, an isopentyl group, a neopentyl group, a 1-methylbutyl group, a 2-methylbutyl group, a hexyl group and the like.

The term "halogen atom" includes a fluorine atom, a chlorine atom, a bromine atom and an iodine atom, the term "lower acyl group" means a straight-chained or branched alkanoyl group having 1 to 6 carbon atoms such as a formyl group, an acetyl group, a propionyl group, a butyryl group, an isobutyryl group, a valeryl group, a hexanoyl group and the like, and the term "halo(lower acyl) group" means a straight-chained or branched alkanoyl group mono-, di- or tri-substituted by same or different halogen atoms as mentioned above and having 2 to 6 carbon atoms such as a trifluoroacetyl group and the like.

The compounds represented by the above general formula (I) of the present invention can be prepared, for example, by converting a 3-cyanobenzenesulfonamide derivative represented by the above general formula:

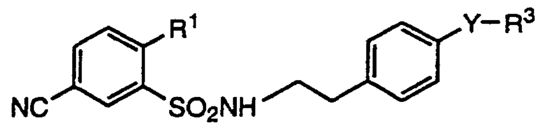

(II)

wherein $R^1$, $R^3$ and Y have the same meanings as mentioned above, into a 3-amidinobenzenesulfonamide derivative represented by the general formula:

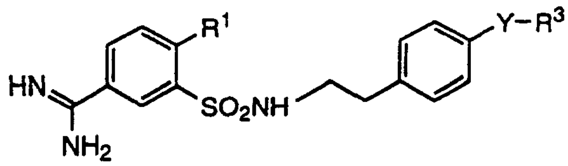

(Ia)

wherein $R^1$, $R^3$ and Y have the same meanings as mentioned above, if necessary, removing the amino-protective group, and, if desired, allowing the resulting compound to react with an alkyliminoether represented by the general formula:

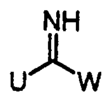

(III)

wherein U represents a lower alkoxy group; and W has the same meaning as mentioned above, a lower acyl halide, a lower carboxylic anhydride, a halo(lower acyl) halide or a halo(lower carboxylic) anhydride.

In the aforementioned production process, as the amino-protective group, the groups commonly used for the protection of an amino group can be used with no particular limitation, and as examples of the protective groups, a carbamate-type group such as tert-butoxycarbonyl and benzyloxycarbonyl, an alkyl-type group such as benzyl and methyl, and an amide-type group such as acetyl, benzoyl and trifluoroacetyl can be illustrated.

The compounds having a phenolic hydroxy group can be allowed to react after introducing an appropriate protective group as occasion demands. As the protective group, the groups commonly used for the protection of the hydroxy group can be used with no particular limitation, and as examples of the protective groups, a silyl ether-type group such as trimethylsilyl and tert-butyl-dimethylsilyl, an ether-type group such as methyl, methoxymethyl, tetrahydropyranyl and benzyl, and an ester-type group such as acetyl and benzoyl can be illustrated.

The reactions from a 3-cyanobenzenesulfonamide derivative represented by the above general formula (II) to a 3-amidinobenzenesulfonamide derivative represented by the above general formula (Ia) in the aforementioned production process are shown as the following scheme:

Scheme 1

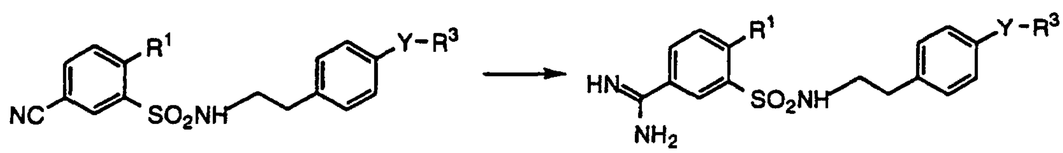

(II) (Ia)

wherein $R^1$, $R^3$ and Y have the same meanings as mentioned above.

Method 1

A 3-cyanobenzenesulfonamide derivative represented by the above general formula (II) is allowed to react with an alcohol such as methanol or ethanol in the presence of hydrogen halide such as hydrogen chloride or hydrogen bromide commonly at from -20 °C to room temperature, and then the resulting imidate compound is allowed to react with ammonia or an ammonium salt such as ammonium carbonate, ammonium chloride or ammonium acetate to obtain

a 3-amidinobenzenesulfonamide derivative represented by the above general formula (Ia). As the solvent used, methanol, ethanol, the alcohol(s) mixed with tetrahydrofuran or dichloromethane, and the like can be illustrated.

Method 2

A 3-cyanobenzenesulfonamide derivative represented by the above general formula (II) is allowed to react with hydrogen sulfide in the presence of an organic base such as triethylamine or pyridine commonly at from -20 °C to room temperature, the resulting thioamide compound is allowed to react with a lower alkyl halide compound such as methyl iodide or ethyl iodide to prepare a thioimidate compound, and then the resulting compound is allowed to react with ammonia or an ammonium salt such as ammonium carbonate, ammonium chloride or ammonium acetate to obtain a 3-amidinobenzenesulfonamide derivative represented by the above general formula (Ia). As the solvent used, methanol, ethanol, tetrahydrofuran, dichloromethane and the like can be illustrated.

When the amino-protective group is not eliminated during the aforementioned reactions, the protective group can be easily removed by treating in the usual way.

The reaction of a cyclic amine compound represented by the general formula (Ia') with an alkyliminoether represented by the above general formula (III), a lower acyl halide, a lower carboxylic anhydride, a halo(lower acyl) halide or a halo(lower carboxylic) anhydride in the aforementioned production process is shown as the following scheme:

Scheme 2

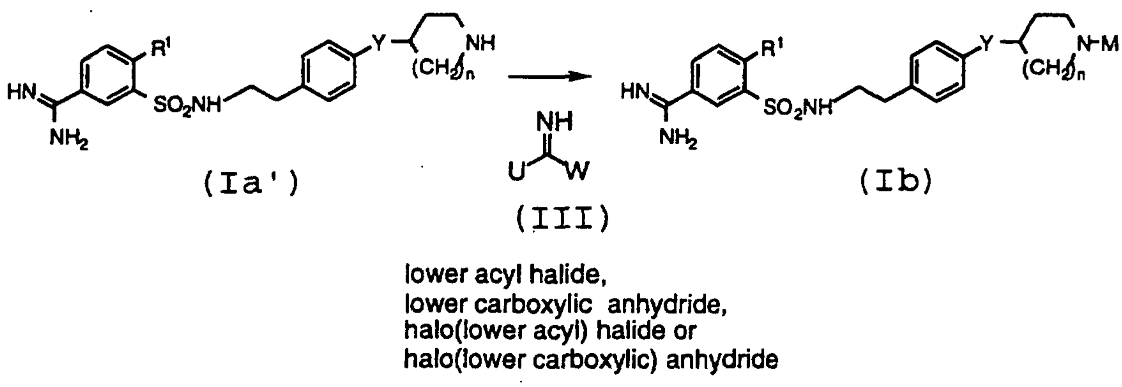


(Ia') (III) (Ib)

wherein M represents a lower acyl group, a halo (lower acyl) group or a group represented by the general formula:

$$-C(=NH)-W$$

(wherein W has the same meaning as mentioned above); and $R^1$, U, W, Y and n have the same meanings as mentioned above.

A compound represented by the above general formula (Ia') is allowed to react with an alkyliminoether represented by the above general formula (III), a lower acyl halide, a lower carboxylic anhydride, a halo(lower acyl) halide or a halo(lower carboxylic) anhydride in the presence of a base such as triethylamine, N-methylmorpholine or pyridine commonly at from -20 °C to 50 °C to obtain a compound represented by the above general formula (Ib). As the solvent used, methanol, ethanol, tetrahydrofuran, dichloromethane, N,N-dimethylformamide, a mixed solvent thereof and the like can be illustrated.

The 3-cyanobenzenesulfonamide derivatives represented by the above general formula (II) which are used as starting materials in the aforementioned production process can be prepared, for example, by the following schemes.

Scheme A

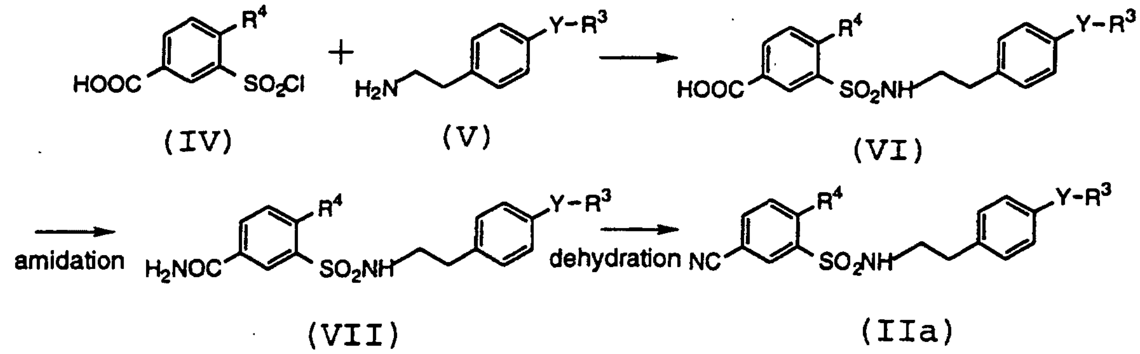


(IV) (V) (VI) (VII) (IIa)

(wherein $R^4$ represents a hydrogen atom or a protected hydroxy group; and $R^3$ and Y have the same meanings as mentioned above)

A 3-carboxybenzenesulfonyl chloride derivative represented by the above general formula (IV) is condensed with a phenethylamine derivative represented by the above general formula (V) in the presence of a base such as triethylamine in a solvent such as dichloromethane, tetrahydrofuran or water, or a mixed solvent thereof commonly at from 0 °C to room temperature to obtain a 3-carboxybenzenesulfonamide derivative represented by the above general formula (VI).

A 3-carbamoylbenzenesulfonamide derivative represented by the above general formula (VII) is derived by allowing the resulting 3-carboxybenzenesulfonamide derivative represented by the above general formula (VI) to react with thionyl chloride in the presence or absence of an activating agent such as N,N-dimethylformamide in an inert solvent such as dichloromethane or without any solvent commonly at from 0 °C to room temperature and subjecting the resulting acyl chloride to amidation with aqueous ammonia, or by, alternatively, subjecting the resulting 3-carboxybenzenesulfonamide derivative represented by the above general formula (VI) to amidation with ammonium chloride using a condensing agent such as 1-ethyl(3-dimethylaminopropyl)carbodiimide hydrochloride and an agent for making an activated ester such as 1-hydroxybenzotriazole in the presence of a base such as triethylamine in an aprotic solvent such as N,N-dimethylformamide commonly at from 0 °C to room temperature. Thereafter, the obtained compound represented by the above general formula (VII) is subjected to dehydration using an acid anhydride such as trifluoroacetic anhydride or a sulfonyl chloride such as tosyl chloride in the presence of a base such as triethylamine or pyridine in an aprotic solvent such as dichloromethane or tetrahydrofuran, or without any solvent commonly at from 0 to 100 °C to obtain a 3-cyanobenzenesulfonamide derivative represented by the above general formula (IIa).

Scheme B

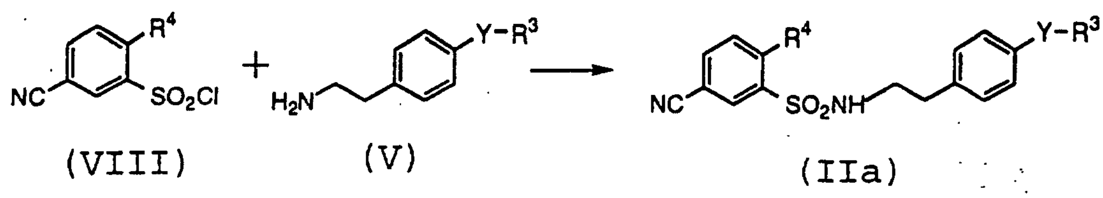

(VIII) (V) (IIa)

(wherein $R^3$, $R^4$ and Y have the same meanings as mentioned above)

A phenethylamine derivative represented by the above general formula (V) is condensed with a 3-cyanobenzenesulfonyl chloride represented by the above general formula (VIII) in the presence of a base such as triethylamine in a solvent such as dichloromethane, tetrahydrofuran or water, or a mixed solvent thereof commonly at from 0 °C to room temperature to obtain a 3-cyanobenzenesulfonamide derivative represented by the above general formula (IIa).

Scheme C

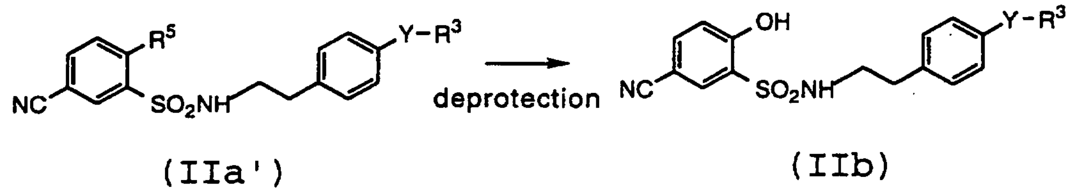


(wherein $R^5$ represents a protected hydroxy group; and $R^3$ and Y have the same meanings as mentioned above)

A 3-cyanobenzenesulfonamide derivative represented by the above general formula (IIa') obtained by the aforementioned Scheme A or B is converted into a 3-cyanobenzenesulfonamide derivative represented by the above general formula (IIb) by deprotection in the usual way. For example, a compound represented by the above general formula (IIa') wherein $R^5$ is a methoxy group is heated in the presence of lithium chloride in a solvent such as N,N-dimethylformamide or N,N-dimethylacetamide commonly at from 100 °C to reflux temperature to obtain the corresponding compound represented by the above general formula (IIb).

The phenethylamine derivatives represented by the above general formula (V) which are used in the aforementioned Scheme A or B can be prepared, for example, by the following Scheme D, E or F.

Scheme D

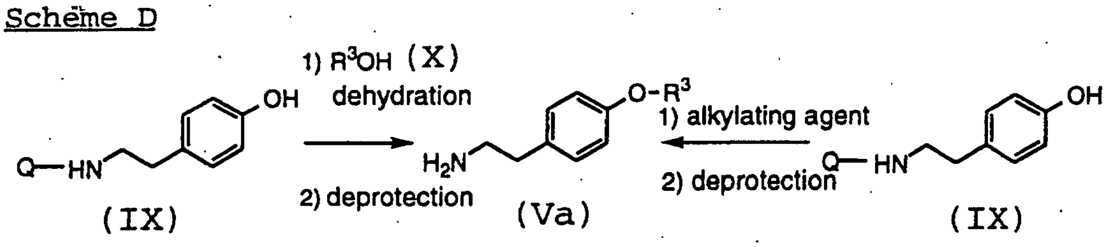


(wherein Q represents an amino-protective group; and $R^3$ has the same meaning as mentioned above)

A compound represented by the above general formula (Va) wherein Y is an oxygen atom in the compound represented by the above general formula (V) can be obtained by subjecting a 4-hydroxyphenethylamine compound represented by the above general formula (IX) to dehydration using an appropriate alcohol represented by the above general formula (X) in the presence of a Mitsunobu reagent in an aprotic solvent such as dichloromethane or tetrahydrofuran commonly at from 0 °C to room temperature, or by, alternatively, subjecting a 4-hydroxyphenethylamine compound represented by the above general formula (IX) to O-alkylation with an appropriate alkylating agent such as an alkyl halide or an alkyl sulfonate in the presence of a base such as sodium hydroxide, potassium carbonate or sodium hydride in a polar solvent such as methanol, ethanol, N,N-dimethylformamide or dimethyl-sulfoxide commonly at from 0 to 100 °C, and then removing the amino-protective group in the usual way.

Scheme E

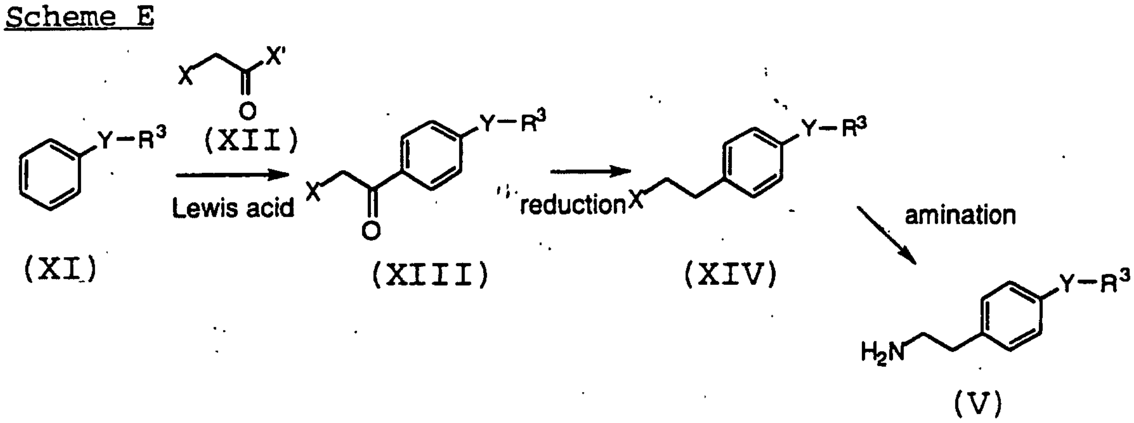

Scheme F

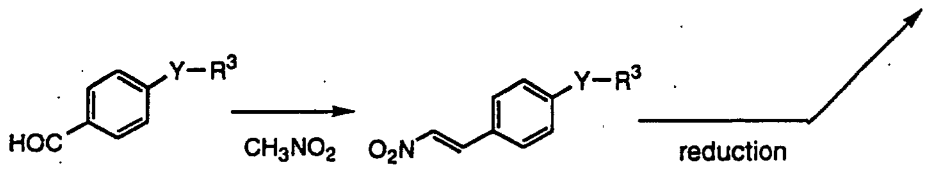


(XV) (XVI)

(wherein X and X' represent a halogen atom; and $R^3$ and Y have the same meanings as mentioned above)

Scheme E

A benzene derivative represented by the above general formula (XI) is allowed to react with a halogenated acyl halide compound represented by the above general formula (XII) such as bromoacetyl bromide in the presence of a Lewis acid such as alminum chloride in an inert solvent such as nitrobenzene, dichloromethane or dichloroethane commonly at from 0 to 100 °C. The resulting p-substituted phenacyl halide derivative represented by the above general formula (XIII) is converted into a p-substituted phenethyl halide derivative represented by the above general formula (XIV) using a reducing agent such as triethylsilane. Thereafter, the resulting compound is allowed to react with an imide-type aminating agent such as potassium phthalimide or sodium formimide, and then the protective group is removed in the usual way to obtain a compound represented by the above general formula (V).

Scheme F

A benzaldehyde derivative represented by the above general formula (XV) is subjected to dehydration in the presence of ammonium acetate in nitromethane commonly at from 50 °C to reflux temperature. The resulting nitroolefin derivative represented by the above general formula (XVI) is subjected to reduction using a hydride-type reducing agent such as lithium aluminum hydride or to catalytic hydrogenation using platinum oxide or the like under a hydrogen atmosphere to obtain a compound represented by the above general formula (V).

The compounds of the present invention obtained by the aforementioned production process can be readily isolated and purified by conventional separation means such as fractional crystallization, precipitation, purification using column chromatography, solvent extraction and the like.

The 3-amidinobenzenesulfonamide derivatives represented by the above general formula (I) of the present invention can be converted into their pharmaceutically acceptable salts in the usual way. Examples of the such salts include acid addition salts with mineral acids (e.g., hydrochloric acid, hydrobromic acid, hydroiodic acid, sulfuric acid, nitric acid, phosphoric acid and the like), acid addition salts with organic acids (e.g., formic acid, acetic acid, methanesulfonic acid, benzenesulfonic acid, p-toluenesulfonic acid, propionic acid, citric acid, succinic acid, tartaric acid, fumaric acid, butyric acid, oxalic acid, malonic acid, maleic acid, lactic acid, malic acid, carbonic acid, glutamic acid, aspartic acid and the like), and salts with inorganic bases such as a sodium salt, a potassium salt and a calcium salt.

In addition, the compounds represented by the above general formula (I) of the present invention also include their hydrates and solvates with pharmaceutically acceptable solvents (e.g., ethanol).

Of the compounds represented by the above general formula (I) of the present invention, compounds having an asymmetric carbon atom exist in two optical isomer forms of (*R*) configuration and (*S*) configuration. Either one of the isomers or a mixture thereof can be employed in the present invention.

The compounds represented by the above general formula (I) of the present invention are compounds having a potent inhibitory activity on activated blood coagulation factor X and anticoagulation activity. The compounds represented by the above general formula (I) of the present invention also have an extremely weak inhibitory activity on thrombin and therefore are highly selective activated blood coagulation factor X inhibitors.

The compounds represented by the above general formula (I) of the present invention are selective activated blood coagulation factor X inhibitors. In consequence, the compounds of the present compounds are extremely useful as agents for the prevention or treatment of cerebral infarction, cerebral thrombosis, cerebral embolism, transient cerebral ischemic attack (TIA), subarachnoid hemorrhage, myocardial infarction, unstable angina, pulmonary thrombosis, pulmonary embolism, Berger disease, peripheral arterial obstruction, deep venous thrombosis, disseminated intravascular coagulation syndrome, thrombus formation after artificial blood vessel operation or after artificial valve replacement, restenosis and reocculusion after coronary intervention such as percutaneous transluminal coronary angioplasty (PTCA) or percutaneous transluminal coronary recanalization (PTCR) surgery, thrombus formation at the

time of extracorporeal circulation and the like, agents for the prevention of blood coagulation at the time of insertion of blood vessel catheters, and agents for the prevention or treatment of influenza virus infection based on the activity to inhibit growth of influenza virus.

The compounds represented by the above general formula (I) of the present invention are highly safe compounds. For example, in acute toxicity tests using rats, no death was observed after an intravenous administration of 5-amidino-2-hydroxy-N-[2-[4-[1-(1-iminoethyl)piperidin-4-yl]phenyl]ethyl]benzene-sulfonamide dihydrochloride at a dose of 30 mg/kg.

When the 3-amidinobenzenesulfonamide derivatives represented by the above general formula (I) of the present invention and pharmaceutically acceptable salts thereof are employed in practical treatment, they are administered orally. or parenterally in the form of appropriate pharmaceutical compositions such as tablets, powders, fine granules, granules, capsules, injections, solutions, adhesive preparations, ointments, suppositories and the like. These pharmaceutical compositions can be formulated in accordance with conventional methods using conventional pharmaceutical carriers, excipients and other additives.

The dosage is appropriately decided depending on the sex, age, body weight, degree of symptoms and the like of each patient to be treated, which is approximately within the range of from 1 to 5,000 mg per day per adult human in the case of oral administration and approximately within the range of from 0.01 to 500 mg per day per adult human in the case of parenteral administration, and the daily dose can be divided into one to several doses per day.

## Industrial Applicability

The compounds of the present invention have a potent and selective inhibitory activity on activated blood coagulation factor X, and are therefore extremely suitable for the prevention or treatment of thromboembolic diseases.

## Best Mode for Carrying Out the Invention

The present invention is further illustrated in more detail by way of the following Reference Examples, Examples and Test Examples. The present invention is not limited thereto.

### Reference Example 1

4-[4-(2-Bromoacetyl)phenyl]-1-trifluoroacetylpiperidine

Aluminum chloride (122 g) and 50 mL of bromoacetyl bromide were added successively to 1.0 L of dichloromethane under an argon atmosphere at 0 °C. To the mixture was added slowly a solution of 118 g of 4-phenyl-1-trifluoroacetylpiperidine in 200 mL of dichloromethane at 0 °C. After being stirred at 0°C for 2 hours, the reaction mixture was poured onto ice and extracted with dichloromethane. The organic layer was washed with aqueous sodium bicarbonate solution and brine, and dried over anhydrous magnesium sulfate. The solvent was removed under reduced pressure, and the resulting residue was dissolved in diethyl ether. The resulting solution was washed with aqueous sodium bicarbonate solution and brine, and dried over anhydrous magnesium sulfate. The solvent was removed under reduced pressure to give 167 g of 4-[4-(2-bromoacetyl)phenyl]-1-trifluoroacetylpiperidine.
$^1$H-NMR($CDCl_3$) $\delta$ ppm:
1.65-1.82 (2H, m), 1.92-2.08 (2H, m), 2.80-2.99 (2H, m), 3.19-3.33 (1H, m), 4.10-4.24 (1H, m), 4.43 (2H, s), 4.67-4.80 (1H, m), 7.33 (2H, d, J=8.4Hz), 7.96 (2H, d, J=8.4Hz)

### Reference Example 2

4-[4-(2-Bromoethyl)phenyl]-1-trifluoroacetylpiperidine

To a solution of 48.3 g of 4-[4-(2-bromoacetyl)-phenyl]-1-trifluoroacetylpiperidine in 200 mL of trifluoro-acetic acid was added dropwise 62 mL of triethylsilane. After being stirred at 50 °C for 2 hours, the reaction mixture was concentrated under reduced pressure. The resulting residue was dissolved in diethyl ether, washed successively with water, aqueous sodium bicarbonate solution and brine, and dried over anhydrous magnesium sulfate. The solvent was removed under reduced pressure, and then n-hexane was added to the resulting residue. The generating precipitates were collected by filtration and washed with n-hexane to give 10.2 g of 4-[4-(2-bromoethyl)phenyl]-1-trifluoroacetylpiperidine.
$^1$H-NMR($CDCl_3$) $\delta$ ppm:
1.62-1.78 (2H, m), 1.91-2.03 (2H, m), 2.74-2.91 (2H, m), 3.15 (2H, t, J=7.6Hz), 3.18-3.30 (1H, m), 3.56 (2H, t, J=7.6Hz), 4.08-4.20 (1H, m), 4.64-4.75 (1H, m), 7.10-7.22 (4H, m)

Reference Example 3

4-[4-(2-Aminoethyl)phenyl]-1-trifluoroacetylpiperidine hydrochloride

Sodium (14.5 g) was added to 250 mL of methanol under an argon atmosphere at 0 °C, and the mixture was stirred at room temperature for 2 hours. To the solution was added dropwise 50 mL of formamide, and the mixture was stirred at room temperature for 10 minutes. After the reaction mixture was stirred under weak aspiration at 80 °C for 1 hour, the solvent was removed under reduced pressure to give 60.3 g of sodium formimide. 4-[4-(2-Bromoethyl) phenyl]-1-trifluoroacetylpiperidine (100.2 g) and 40 g of sodium formimide were added to 300 mL of N,N-dimethylformamide, and the mixture was stirred under an argon atmosphere at 120 °C for 5 hours. To the reaction mixture was added 800 mL of diethyl ether, then the insoluble material was filtered off through Celite®, and the filtrate was washed successively with 10 % aqueous citric acid solution, aqueous sodium bicarbonate solution and brine, and dried over anhydrous magnesium sulfate. After the solvent was removed under reduced pressure, 400 mL of ethanol and 100 mL of 30 % hydrogen chloride ethanol solution were added to the redisue, and the mixture was stirred at 50°C for 3 hours. After the solvent was removed under reduced pressure, the resulting residue was suspended in 600 mL ofdiethyl ether, and the precipitates were collected by filtration and washed with diethyl ether to give 52.6 g of 4-[4-(2-aminoethyl)phenyl]-1-trifluoroacetylpiperidine hydrochloride.
$^1$H-NMR(DMSO-$d_6$) δ ppm:
1.50-1.70 (2H, m), 1.78-1.95 (2H, m), 2.70-3.06 (6H, m), 3.87-4.01 (1H, m), 4.36-4.48 (1H, m), 7.10-7.29 (4H, m), 8.08 (3H, br-s)

Reference Example 4

1-Isopropyl-4-(2-nitrovinyl)benzene

4-Isopropylbenzaldehyde (5.0 mL) and 266 mg of ammonium acetate were suspended in 30 mL of nitromethane, and the mixture was heated under reflux for 1.5 hours with azeotropic removal of water. The solvent was removed under reduced pressure, and the resulting residue was purified by silica gel column chromatography (eluent: ethyl acetate-hexane) to give 2.26 g of l-isopropyl-4-(2-nitrovinyl)benzene.
$^1$H-NMR($CDCl_3$) δ ppm:
1.27 (6H, d, J=6.9Hz), 2.87-3.03 (1H, m), 7.31 (2H, d, J=8.2Hz), 7.49 (2H, d, J=8.2Hz), 7.58 (1H, d, J=13.6Hz), 8.00 (1H, d, J=13.6Hz)

Reference Example 5

2-(4-Isopropylphenyl)ethylamine

Lithium aluminum hydride (900 mg) was suspended in 30 mL of anhydrous diethyl ether under ice-cooling with stirring. A solution of 2.26 g of l-isopropyl-4-(2-nitrovinyl)benzene in 20 mL of anhydrous diethyl ether was added dropwise to the suspension, and the mixture was stirred at room temperature for 1 hour. An additional lithium aluminum hydride (224 mg) was added to the reaction mixture. After the mixture was stirred at room temperature for 1 hour, 60 mL of 2 mol/L aqueous sodium hydroxide solution was added slowly to the reaction mixture under ice-cooling. To the mixture was added 50 mL of diethyl ether, and then the resulting mixture was filtered through Celite®. The filtrate was extracted with diethyl ether, and the organic layer was extracted with dilute hydrochloric acid. The aqueous layer was alkalized with 2 mol/L aqueous sodium hydroxide solution and extracted with diethyl ether. The organic layer was washed with brine and dried over anhydrous magnesium sulfate, and the solvent was removed under reduced pressure to give 1.47 g of 2-(4-isopropylphenyl)ethylamine.
$^1$H-NMR($CDCl_3$) δ ppm:
1.24 (6H, d, J=6.9Hz), 2.66-3.02 (5H, m), 7.07-7.22 (4H, m)

Reference Example 6

N-tert-Butoxycarbonyl-2-(4-hydroxyphenyl)ethylamine

Tyramine (3.0 g) was suspended in 30 mL of dichloromethane. To the suspension were added successively 3.36 mL of triethylamine and 5.01 g of di-tert-butyl dicarbonate with stirring at room temperature. After the mixture was stirred at room temperature for 1 day, water was added to the reaction mixture, and the mixture was extracted with diethyl ether. The organic layer was washed successively with 10 % aqueous citric acid solution and brine, and dried

over anhydrous magnesium sulfate. The solvent was removed under reduced pressure to give 5.74 g of N-tert-butoxycarbonyl-2-(4-hydroxyphenyl)ethylamine.
$^{1}$H-NMR($CDCl_3$) δ ppm:
1.44 (9H, s), 2.71 (2H, t, J=7.0Hz), 3.23-3.41 (2H, m), 4.50-4.64 (1H, m), 5.43 (1H, br), 6.77 (2H, d, J=8.4Hz), 7.07 (2H, d, J=8.4Hz)

Reference Example 7

N-tert-Butoxycarbonyl-2-(4-isopropoxyphenyl)ethylamine

N-tert-Butoxycarbonyl-2-(4-hydroxyphenyl)ethylamine (4.0 g) and 3.5 g of potassium carbonate were dissolved in 15 mL of N,N-dimethylformamide. To the solution was added 2.0 mL of 2-iodopropane at room temperature with stirring, and the mixture was stirred at 80 °C for 1 hour. To the reaction mixture was added diethyl ether, and the mixture was filtered through Celite®. The filtrate was washed with water and brine, and dried over anhydrous magnesium sulfate. After the solvent was removed under reduced pressure, the resulting residue was purified by silica gel column chromatography (eluent: ethyl acetate-hexane) to give 3.0 g of N-tert-butoxycarbonyl-2-(4-isopropoxyphenyl) ethylamine.
$^{1}$H-NMR($CDCl_3$) δ ppm:
1.32 (6H, d, J=6.0Hz), 1.43 (9H, s), 2.65-2.82 (2H, m), 3.25-3.45 (2H, m), 4.42-4.63 (2H, m), 6.82 (2H, d, J=8.4Hz), 7.08 (2H, d, J=8.4Hz)

Reference Example 8

2-(4-Isopropoxyphenyl)ethylamine

N-tert-Butoxycarbonyl-2-(4-isopropoxyphenyl)ethylamine (2.5 g) was dissolved in 10 mL of dichloromethane. Trifluoroacetic acid (2.07 mL) was added to the solution with stirring at room temperature. After the mixture was stirred at 40 °C for 40 minutes, the solvent was removed under reduced pressure. To the resulting residue was added dilute hydrochloric acid, and the mixture was washed with diethyl ether. The aqueous layer was alkalized with 2 mol/L aqueous sodium hydroxide solution and extracted with dichloromethane. The organic layer was washed with brine and dried over anhydrous magnesium sulfate. The solvent was removed under reduced pressure to give 1.16 g of 2-(4-isopropoxyphenyl)ethylamine.
$^{1}$H-NMR($CDCl_3$) δ ppm:
1.32 (6H, d, J=6.0Hz), 2.68 (2H, t, J=6.8Hz), 2.88-2.99 (2H, m), 4.46-4.58 (1H, m), 6.83 (2H, d, J=8.6Hz), 7.09 (2H, d, J=8.6Hz)

Reference Example 9

N-Benzyloxycarbonyl-2-(4-hydroxyphenyl)ethylamine

Tyramine (3.0 g) was suspended in 30 mL of dichloromethane. To the suspension were added successively 3.36 mL of triethylamine, 2.97 mL of benzyl chloroformate and 20 mL of N,N-dimethylformamide with stirring at room temperature. After the mixture was stirred at room temperature for 1 day, dilute hydrochloric acid was added to the reaction mixture, and the mixture was extracted with diethyl ether. The organic layer was washed successively with dilute hydrochloric acid, aqueous sodium bicarbonate solution and brine, and dried over anhydrous magnesium sulfate. After the solvent was removed under reduced pressure, the resulting residue was purified by silica gel column chromatography (eluent: dichloromethane-diethyl ether) to give 1.32 g of N-benzyloxycarbonyl-2-(4-hydroxyphenyl) ethylamine.
$^{1}$H-NMR($CDCl_3$) δ ppm:
2.74 (2H, t, J=6.8Hz), 3.33-3.52 (2H, m), 4.69-4.84 (1H, m), 5.03-5.25 (3H, m), 6.75 (2H, d, J=8.3Hz), 7.02 (2H, d, J=8.3Hz), 7.24-7.43 (5H, m)

Reference Example 10

N-Benzyloxycarbonyl-2-[4-(1-tert-butoxycarbonylpiperidin-4-yloxy)phenyl]ethylamine

N-Benzyloxycarbonyl-2-(4-hydroxyphenyl)ethylamine (716 mg), 531 mg of 1-tert-butoxycarbonyl-4-hydroxypiperidine and 1.04 g of triphenylphosphine were dissolved in 5 mL of dichloromethane. Diethyl azodicaboxylate (0.624

mL) was added slowly dropwise to the solution at room temperature with stirring. After the mixture was stirred at room temperature for 1 day, 10 % aqueous citric acid solution was added to the reaction mixture, and the mixture was extracted with diethyl ether. The organic layer was washed successively with aqueous sodium bicarbonate solution and brine, and dried over anhydrous magnesium sulfate. After the solvent was removed under reduced pressure, the resulting residue was purified by silica gel column chromatography (eluent: dichloromethane-diethyl ether) to give 718 mg of N-benzyloxycarbonyl-2-[4-(1-tert-butoxycarbonylpiperidin-4-yloxy)phenyl]ethylamine.
$^1$H-NMR ($CDCl_3$) δ ppm:
1.47 (9H, s), 1.67-1.80 (2H, m), 1.85-1.96 (2H, m), 2.75 (2H, t, J=6.8Hz), 3.28-3.49 (4H, m), 3.65-3.76 (2H, m), 4.38-4.48 (1H, m), 4.68-4.79 (1H, m), 5.09 (2H, s), 6.84 (2H, d, J=8.5Hz), 7.08 (2H, d, J=8.5Hz), 7.27-7.41 (5H, m)

Reference Example 11

The following compound was prepared in a similar manner to that described in Reference Example 10.

(S)-3-[4-(2-Benzyloxycarbonylaminoethyl)phenoxy]-1-tert-butoxycarbonylpyrrolidine

$^1$H-NMR(DMSO-$d_6$) δ ppm:
1.30-1.45 (9H, m), 1.90-2.20 (2H, m), 2.58-2.70 (2H, m), 3.10-3.60 (6H, m), 4.88-5.05 (3H, m), 6.84 (2H, d, J=8.6Hz), 7.11 (2H, d, J=8.6Hz), 7.22-7.40 (5H, m)

Reference Example 12

2-[4-(1-tert-Butoxycarbonylpiperidin-4-yloxy)phenyl]ethyl-amine

N-Benzyloxycarbonyl-2-[4-(1-tert-butoxycarbonyl-piperidin-4-yloxy)phenyl]ethylamine (1.16 g) was dissolved in a mixture of 5 mL of ethanol and 5 mL of tetrahydrofuran. To the solution was added 0.23 g of 10 % palladium on carbon catalyst (wet), and the mixture was stirred under a hydrogen atmosphere at room temperature for 4 hours. After the insoluble material was filtered off through Celite®, the solvent of the filtrate was removed under reduced pressure to give 0.81 g of 2-[4-(1-tert-butoxycarbonylpiperidin-4-yloxy)phenyl]ethylamine.
$^1$H-NMR($CDCl_3$) δ ppm:
1.47 (9H, s), 1.68-1.82 (2H, m), 1.84-1.98 (2H, m), 2.67 (2H, t, J=6.8Hz), 2.93 (2H, t, J=6.8Hz), 3.25-3.40 (2H, m), 3.62-3.81 (2H, m), 4.38-4.48 (1H, m), 6.85 (2H, d, J=8.5Hz), 7.10 (2H, d, J=8.5Hz)

Reference Example 13

The following compound was prepared in a similar manner to that described in Reference Example 12.

(S)-2-[4-(1-tert-Butoxycabonylpyrrolidin-3-yloxy)phenyl]-ethylamine

$^1$H-NMR(DMSO-$d_6$) δ ppm:
1.39 (9H, s), 1.91-2.20 (2H, m), 2.45-2.87 (4H, m), 4.86-5.00 (1H, m), 6.84 (2H, d, J=8.2Hz), 7.11 (2H, d, J=8.2Hz)

Reference Example 14

3-Chlorosulfonvl-4-methoxvbenzoic acid

Ethyl 4-methoxybenzoate (1.35 mL) was added slowly to 4 mL of chlorosulfonic acid under an argon atmosphere under ice-cooling. After being stirred at 80°C for 1 hour, the mixture was poured into ice-water. After the precipitates were collected by filtration, the resulting solid was washed successively with water and hexane, and dried to give 1.2 g of 3-chlorosulfonyl-4-methoxybenzoic acid.
$^1$H-NMR (DMSO-$d_6$) δ ppm:
3.83 (3H, s), 7.06 (1H, d, J=8.6Hz), 7.90 (1H, dd, J=8.6Hz, 2.4Hz), 8.30(1H, d, J=2.4Hz), 13.78 (1H, br-s)

Reference Example 15

3-[N-[2-(4-Isopropylphenyl)ethyl]sulfamoyl]-4-methoxybenzoic acid

2-(4-Isopropylphenyl)ethylamine (1.2 g) and 2.1 mL of triethylamine were dissolved in 15 mL of dichloromethane. To the solution was added a solution of 1.5 g of 3-chlorosulfonyl-4-methoxybenzoic acid in 10 mL of dichloromethane under ice-cooling over 10 minute period. After the mixture was stirred at room temperature for 3 hours, dilute hydrochloric acid was added to the reaction mixture, and the mixture was extracted with dichloromethane. The organic layer was washed with brine and dried over anhydrous magnesium sulfate. After the solvent was removed under reduced pressure, the resulting residue was suspended with a small amount of diethyl ether. The resulting precipitates were collected by filtration to give 1.2 g of 3-[N-[2-(4-isopropylphenyl)ethyl]sulfamoyl]-4-methoxybenzoic acid.
$^{1}$H-NMR ($CDCl_3$) δ ppm:
1.23 (6H, d, J=6.9Hz), 2.68-3.00 (3H, m), 3.10-3.25 (2H, m), 3.71 (3H, s), 4.78-4.81 (1H, m), 6.95-7.06 (3H, m), 7.15 (2H, d, J=8.0Hz), 8.27 (1H, dd, J=8.7Hz, 2.2Hz), 8.65 (1H, d, J=2.2Hz)

Reference Example 16

The following compound was prepared in a similar manner to that described in Reference Example 15.

3-[N-[2-(4-Isopropoxyphenyl)ethyl]sulfamoyl]-4-methoxy-benzoic acid

$^{1}$H-NMR($CDCl_3$) δ ppm:
1.32 (6H, d, J=6.0Hz), 2.73 (2H, t, J=6.5Hz), 3.10-3.20 (2H, m), 3.76 (3H, s), 4.43-4.57 (1H, m), 4.76-4.85 (1H, m), 6.80 (2H, d, J=8.6Hz), 6.93-7.05 (3H, m), 8.27 (1H, dd, J=8.7Hz, 2.2Hz), 8.64 (1H, d, J=2.2Hz)

Reference Example 17

3-[N-[2-[4-(1-tert-Butoxycarbonylpiperidin-4-yloxy)phenyl]-ethyl]sulfamoyl]-4-methoxybenzoic acid

2-[4-(1-tert-Butoxycarbonylpiperidin-4-yloxy)phenyl]-ethylamine (1.3 g) and 0.6 mL of triethylamine were dissolved in a mixture of 20 mL of tetrahydrofuran and 10 mL of water. 3-Chlorosulfonyl-4-methoxybenzoic acid (1.0 g) was added portionwise to the solution. After the mixture was stirred at room temperature for 1.5 hours, dilute hydrochloric acid was added to the reaction mixture, and the mixture was extracted with ethyl acetate. The organic layer was washed with brine and dried over anhydrous magnesium sulfate. The solvent was removed under reduced pressure to give 1.6 g of 3-[N-[2-[4-(1-tert-butoxy-carbonylpiperidin-4-yloxy)phenyl]ethyl]sulfamoyl]-4-methoxy-benzoic acid.
$^{1}$H-NMR($CDCl_3$) δ ppm:
1.47 (9H, s), 1.65-1.80 (2H, m), 1.85-1.98 (2H, m), 2.74 (2H, t, J=6.5Hz), 3.10-3.21 (2H, m), 3.28-3.40 (2H, m), 3.64-3.76 (2H, m), 3.80 (3H, s), 4.38-4.48 (1H, m), 4.83 (1H, t, J=6.1Hz), 6.82 (2H, d, J=8.6Hz), 6.95-7.08 (3H, m), 8.27 (1H, dd, J=8.7Hz, 2.2Hz), 8.64 (1H, d, J=2.2Hz)

Reference Example 18

The following compounds were prepared in a similar manner to that described in Reference Example 17.

4-Methoxy-3-[N-[2-[4-(1-trifluoroacetylpiperidin-4-yl)-phenyl]ethyl]sulfamoyl]benzoic acid

$^{1}$H-NMR(DMSO-$d_6$) δ ppm:
1.46-1.65 (2H, m), 1.75-1.92 (2H, m), 2.55-3.05 (6H, m), 3.83-4.00 (4H, m), 4.35-4.48 (1H, m), 7.03 (2H, d, J=8.1Hz), 7.12 (2H, d, J=8.1Hz), 7.28 (1H, d, J=8.8Hz), 7.45 (1H, t, J=5.7Hz), 8.12 (1H, dd, J=8.8Hz, 2.2Hz), 8.26 (1H, d, J=2.2Hz), 12.70-13.40 (1H, br)

(S)-3-[N-[2-[4-(1-tert-Butoxycarbonylpyrrolidin-3-yloxy)-phenyl]ethyl]sulfamoyl]-4-methoxybenzoic acid

$^{1}$H-NMR(DMSO-$d_6$) δ ppm:
1.26-1.47 (9H, m), 1.90-2.18 (2H, m), 2.52-2.68 (2H, m), 2.89-3.05 (2H, m), 3.21-3.59 (4H, m), 3.93 (3H, s), 4.84-4.98 (1H, m), 6.79 (2H, d, J=8.6Hz), 7.02 (2H, d, J=8.6Hz), 7.29 (1H, d, J=8.7Hz), 7.44 (1H, t, J=5.7Hz), 8.12 (1H, dd, J=8.7Hz, 2.2Hz), 8.26 (1H, d, J=2.2Hz), 13.05 (1H, br-s) Specific rotation: $[\alpha]_D^{29}$ = +11.7° (c=0.55, methanol)

3-[N-[2-(4-Isopropylphenyl)ethyl]sulfamoyl]benzoic acid

$^{1}$H-NMR(DMSO-$d_6$) δ ppm:
1.16 (6H, d, J=6.9Hz), 2.62 (2H, t, J=7.5Hz), 2.72-3.04 (3H, m), 7.04 (2H, d, J=8.1Hz), 7.11 (2H, d, J=8.1Hz), 7.66-7.77 (1H, m), 7.82-7.91 (1H, m), 7.96-8.06 (1H, m), 8.11-8.21 (1H, m), 8.31 (1H, t, J=1.8Hz), 11.30-11.60 (1H, br)

Reference Example 19

3-[N-[2-(4-Isopropylphenyl)ethyl]sulfamoyl]-4-methoxybenzamide

3-[N-[2-(4-Isopropylphenyl)ethyl]sulfamoyl]-4-methoxybenzoic acid (1.2 g) and 0.7 mL of thionyl chloride were suspended in 15 mL of toluene, and two drops of N,N-dimethylformamide was added to the mixture. After the mixture was stirred at 90 °C for 1 hour, the solvent was removed under reduced pressure. To the resulting residue was added 30 mL of 25 % aqueous ammonia solution, and the mixture was stirred at room temperature for 1 hour. The precipitates were collected by filtration and washed successively with water, diethyl ether and hexane to give 1.1 g of 3-[N-[2-(4-isopropylphenyl)ethyl]sulfamoyl]-4-methoxybenzamide.
$^1$H-NMR($CDCl_3$) δ ppm:
1.23 (6H, d, J=6.9Hz), 2.76 (2H, t, J=6.6Hz), 2.83-2.95 (1H, m), 3.08-3.20 (2H, m), 3.68 (3H, s), 4.86 (1H, t, J=6.2Hz), 5.45-6.50 (2H, m), 6.95-7.05 (3H, m), 7.15 (2H, d, J=8.0Hz), 8.16 (1H, dd, J=8.7Hz, 2.3Hz), 8.24 (1H, d, J=2.3Hz)

Reference Example 20

The following compounds were prepared according to a similar manner to that described in Reference Example 19.

3-[N-[2-(4-Isopropoxyphenyl)ethyl]sulfamoyl]-4-methoxybenzamide

$^1$H-NMR($CDCl_3$) δ ppm:
1.32 (6H, d, J=6.1Hz), 2.73 (2H, t, J=6.5Hz), 3.07-3.18 (2H, m), 3.73 (3H, s), 4.45-4.57 (1H, m), 4.82 (1H, t, J=5.8Hz), 5.35-6.30 (2H, m), 6.80 (2H, d, J=8.6Hz), 6.92-7.06 (3H, m), 8.16 (1H, dd, J=8.7Hz, 2.3Hz), 8.23 (1H, d, J=2.3Hz)

4-Methoxy-3-[N-[2-[4-(1-trifluoroacetylpiperidin-4-yl)-phenyl]ethyl]sulfamoyl]benzamide

$^1$H-NMR(DMSO-$d_6$) δ ppm:
1.48-1.56 (2H, m), 1.78-1.93 (2H, m), 2.56-3.04 (6H, m), 3.83-4.00 (4H, m), 4.35-4.48 (1H, m), 7.03 (2H, d, J=8.1Hz), 7.13 (2H, d, J=8.1Hz), 7.24 (1H, d, J=8.8Hz), 7.28-7.43 (2H, m), 7.98-8.15 (2H, m), 8.26 (1H, d, J=2.3Hz)

3-[N-[2-[4-(1-tert-Butoxycarbonylpiperidin-4-yloxy)phenyl]-ethyl]sulfamoyl]-4-methoxvbenzamide

$^1$H-NMR($CDCl_3$) δ ppm:
1.47 (9H, s), 1.65-1.80 (2H, m), 1.86-1.98 (2H, m), 2.74 (2H, t, J=6.5Hz), 3.08-3.20 (2H, m), 3.28-3.40 (2H, m), 3.65-3.85 (5H, m), 4.39-4.49 (1H, m), 4.78-4.86 (1H, m), 5.35-6.35 (2H, m), 6.82 (2H, d, J=8.5Hz), 6.95-7.08 (3H, m), 8.16 (1H, dd, J=8.7Hz, 2.3Hz), 8.23 (1H, d, J=2.3Hz)

(S)-3-[N-[2-[4-(1-tert-Butoxycarbonylpirrolidin-3-yloxy)-phenyl]ethyl]sulfamoyl]-4-methoxvbenzamide

$^1$H-NMR (DMSO-$d_6$) δ ppm:
1.30-1.46 (9H, m), 1.92-2.17 (2H, m), 2.52-2.66 (2H, m), 2.90-3.03 (2H, m), 3.22-3.58 (4H, m), 3.91 (3H, s), 4.86-4.98 (1H, m), 6.80 (2H, d, J=8.6Hz), 7.02 (2H, d, J=8.6Hz), 7.20-7.40 (3H, m), 7.96-8.14 (2H, m), 8.26 (1H, d, J=2.3Hz)
Specific rotation: $[\alpha]_D^{29}$ = +13.5° (c=0.54, methanol)

3-[N-[2-(4-Isopropylphenyl)ethyl]sulfamoyl]benzamide $^1$H-NMR(DMSO-$d_6$) δ ppm:

1.16 (6H, d, J=6.9Hz), 2.55-2.68 (2H, m), 2.75-3.02 (3H, m), 7.05 (2H, d, J=8.1Hz), 7.12 (2H, d, J=8.1Hz), 7.51-7.72 (2H, m), 7.78 (1H, t, J=5.8Hz), 7.87-7.95 (1H, m), 8.05-8.15 (1H, m), 8.21 (1H, br-s), 8.29 (1H, t, J=1.6Hz)

Reference Example 21

5-Cyano-N-[2-(4-isopropylphenyl)ethyl]-2-methoxybenzene-sulfonamide

3-[N-[2-(4-Isopropylphenyl)ethyl]sulfamoyl]-4-methoxy-benzamide (1.0 g) and 0.65 mL of pyridine were dissolved in 10 mL of dichloromethane. To the solution was added dropwise 1.13 mL of trifluoroacetic anhydride under ice-cooling. After the mixture was stirred at room temperature for 45 minutes, water was added to the reaction mixture, and the mixture was extracted with diethyl ether. The organic layer was washed successively with dilute hydrochloric

acid and brine, and dried over anhydrous magnesium sulfate. The solvent was removed under reduced pressure to give 733 mg of 5-cyano-N-[2-(4-isopropylphenyl)-ethyl]-2-methoxybenzenesulfonamide.
$^1$H-NMR($CDCl_3$) δ ppm:
1.23 (6H, d, J=6.9Hz), 2.76 (2H, t, J=6.4Hz), 2.83-2.95 (1H, m), 3.14-3.25 (2H, m), 3.71 (3H, s), 4.75-4.88 (1H, m), 6.95-7.07 (3H, m), 7.15 (2H, d, J=8.1Hz), 7.80 (1H, dd, J=8.6Hz, 2.1Hz), 8.20 (1H, d, J=2.1Hz)

Reference Example 22

The following compounds were prepared in a similar manner to that described in Reference Example 21.

5-Cyano-N-[2-(4-isopropoxyphenyl)ethyl]-2-methoxybenzene sulfonamide

$^1$H-NMR($CDCl_3$) δ ppm:
1.33 (6H, d, J=6.1Hz), 2.73 (2H, t, J=6.5Hz), 3.09-3.22 (2H, m), 3.75 (3H, s), 4.42-4.60 (1H, m), 4.72-4.84 (1H, m), 6.79 (2H, d, J=8.6Hz), 6.91-7.08 (3H, m), 7.80 (1H, dd, J=8.7Hz, 2.2Hz), 8.19 (1H, d, J=2.2Hz)

5-Cyano-2-methoxy-N-[2-[4-(1-trifluoroacetylpiperidin-4-yl)-phenyl]ethyl]benzensulfonamide

$^1$H-NMR(DMSO-$d_6$) δ ppm:
1.49-1.56 (2H, m), 1.79-1.93 (2H, m), 2.57-2.68 (2H, m), 2.74-3.10 (4H, m), 3.84-4.02 (4H, m), 4.35-4.48 (1H, m), 7.03 (2H, d, J=8.1Hz), 7.12 (2H, d, J=8.1Hz), 7.35 (1H, d, J=8.7Hz), 7.62 (1H, t, J=5.7Hz), 8.03 (1H, d, J=2.2Hz), 8.07 (1H, dd, J=8.7Hz, 2.2Hz)

5-Cyano-N-[2-[4-(1-tert-butoxycarbonylpiperidin-4-yloxy)-phenyl]ethyl]-2-methoxybenzenesulfonamide

$^1$H-NMR($CDCl_3$) δ ppm:
1.47 (9H, s), 1.67-1.80 (2H, m), 1.85-1.98 (2H, m), 2.74 (2H, t, J=6.6Hz), 3.11-3.21 (2H, m), 3.28-3.40 (2H, m), 3.65-3.76 (2H, m), 3.79 (3H, s), 4.39-4.49 (1H, m), 4.77 (1H, t, J=6.0Hz), 6.82 (2H, d, J=8.6Hz), 6.95-7.06 (3H, m), 7.80 (1H, dd, J=8.7Hz, 2.1Hz), 8.19 (1H, d, J=2.1Hz)

(S)-5-Cyano-N-[2-[4-(1-tert-butoxycarbonylpyrrolidin-3-yl- oxy)phenyl]ethyl]-2-methoxybenzenesulfonamide

$^1$H-NMR(DMSO-$d_6$) δ ppm:
1.30-1.47 (9H, m), 1.92-2.18 (2H, m), 2.53-2.68 (2H, m), 2.96-3.11 (2H, m), 3.21-3.60 (4H, m), 3.95 (3H, s), 4.86-4.99 (1H, m), 6.78 (2H, d, J=8.6Hz), 7.02 (2H, d, J=8.6Hz), 7.36 (1H, d, J=8.7Hz), 7.56 (1H, t, J=5.7Hz), 8.01 (1H, d, J=2.2Hz), 8.05 (1H, dd, J=8.8Hz, 2.2Hz)
Specific rotation: $[\alpha]_D^{29}$ = +19.2° (c=0.54, methanol)

3-Cyano-N-[2-(4-isopropylphenyl)ethyl]-N-trifluoroacetyl-benzenesulfonamide

$^1$H-NMR (DMSO-$d_6$) δ ppm:
1.19 (6H, d, J=6.9 Hz), 2.89-2.95 (1H, m), 3.00-3.14 (2H, m), 4.02-4.17 (2H, m), 7.17 (2H, d, J=8.1Hz), 7.23 (2H, d, J=8.1Hz), 7.85-7.95 (1H, m), 8.24-8.43 (2H, m), 8.62 (1H, t, J=1.5Hz)

Reference Example 23

5-Cyano-N-[2-(4-isopropylphenyl)ethyl]-2-hydroxybenzene-sulfonamide

5-Cyano-N-[2-(4-isopropylphenyl)ethyl]-2-methoxybenzenesulfonamide (733 mg) was dissolved in 10 mL of N,N-dimethylformamide. To the solution was added 260 mg of lithium chloride, and the mixture was stirred at 120°C for 9 hours. After the reaction mixture was cooled to room temperature, 10 % aqueous citric acid solution was added, and the mixture was extracted with ethyl acetate. The organic layer was washed with brine and dried over anhydrous magnesium sulfate. The solvent was removed under reduced pressure to give 700 mg of 5-cyano-N-[2-(4-isopropylphenyl)ethyl]-2-hydroxybenzenesulfonamide.
$^1$H-NMR($CDCl_3$) δ ppm:
1.23 (6H, d, J=6.9Hz), 2.77 (2H, t, J=6.7Hz), 2.84-2.96 (1H, m), 3.25-3.35 (2H, m), 4.67-4.78 (1H, m), 7.00 (2H, d, J=8.0Hz), 7.11 (1H, d, J=8.7Hz), 7.16 (2H, d, J=8.0Hz), 7.70 (1H, dd, J=8.7Hz, 2.0Hz), 7.88 (1H, d, J=2.0Hz), 9.27 (1H, br)

Reference Example 24

The following compounds were prepared in a similar manner to that described in Reference Example 23.

5-Cyano-N-[2-(4-isopropoxyphenyl)ethyl]-2-hydroxybenzene-sulfonamide

$^1$H-NMR($CDCl_3$) δ ppm:
1.33 (6H, d, J=6.1Hz), 2.73 (2H, t, J=6.7Hz), 3.19-3.32 (2H, m), 4.46-4.59 (1H, m), 4.61-4.72 (1H, m), 6.80 (2H, d, J=8.6Hz), 6.96 (2H, d, J=8.6Hz), 7.10 (1H, d, J=8.7Hz), 7.69 (1H, dd, J=8.7Hz, 2.1Hz), 7.87 (1H, d, J=2.1Hz), 9.23 (1H, br-s)

5-Cyano-2-hydroxy-N-[2-[4-(1-trifluoroacetylpiperidin-4-yl)phenyl]ethyl]benzenesulfonamide

$^1$H-NMR(DMSO-$d_6$) δ ppm:
1.46-1.67 (2H, m), 1.76-1.96 (2H, m), 2.60-3.10 (6H, m), 3.87-4.01 (1H, m), 4.33-4.50 (1H, m), 6.95-7.25 (5H, m), 7.53 (1H, br-s), 7.87 (1H, dd, J=8.6Hz, 2.1Hz), 7.98 (1H, d, J=2.1Hz), 12.00 (1H, br-s)

5-Cyano-N-[2-[4-(1-tert-butoxycarbonylpiperidin-4-yloxy)-phenyl]ethyl]-2-hydroxybenzenesulfonamide.

$^1$H-NMR($CDCl_3$) δ ppm:
1.47 (9H, s), 1.67-1.81 (2H, m), 1.86-1.98 (2H, m), 2.74 (2H, t, J=6.7Hz), 3.20-3.40 (4H, m), 3.65-3.78 (2H, m), 4.40-4.50 (1H, m), 4.52-4.90 (1H, br), 6.83 (2H, d, J=8.6Hz), 6.99 (2H, d, J=8.6Hz), 7.11 (1H, d, J=8.7Hz), 7.69 (1H, dd, J=8.7Hz, 2.0Hz), 7.81 (1H, d, J=2.0Hz)

(S)-5-Cyano-N-[2-[4-(1-tert-butoxycarbonylpyrrolidin-3-yloxyl)phenyl]ethyl]-2-hydroxybenzenesulfonamide

$^1$H-NMR(DMSO-$d_6$) δ ppm:
1.30-1.45 (9H, m), 1.92-2.19 (2H, m), 2.55-2.67 (2H, m), 2.92-3.05 (2H, m), 4.85-4.98 (1H, m), 6.80 (2H, d, J=8.4Hz), 6.94-7.11 (3H, m), 7.78 (1H, d, J=8.6Hz), 7.91 (1H, s)
Specific rotation: $[\alpha]_D^{29}$ = +17.0 ° (c=0.52, methanol)

Example 1

5-Amidino-2-hydroxy-N-[2-[4-(1-trifluoroacetylpiperidin-4-yl)phenyl]ethyl]benzenesulfonamide (Compound 1)

5-Cyano-2-hydroxy-N-[2-[4-(1-trifluoroacetylpiperidin-4-yl)phenyl]ethyl]benzenesulfonamide (1.964 g) was dissolved in 25 mL of hydrogen chloride ethanol solution, and the reaction flask was sealed. The mixture was stirred at room temperature for 19 hours. After the reaction mixture was concentrated, ethanol was added to the residue. After the solvent was removed under reduced pressure, the resulting residue was suspended in diethyl ether. The supernatant was removed, and the residue was 'dissolved in 20 mL of methanol. Ammonium acetate (1.572 g) was added to the solution, and the mixture was stirred at room temperature for 2.5 days. After the reaction mixture was concentrated, dichloromethane was added to the residue. After the insoluble material was filtered off through Celite®, the filtrate was concentrated. Water was added to the resulting residue, and the resulting precipitates were collected by filtration and washed with water to give 1.726 g of 5-amidino-2-hydroxy-N-[2-[4-(1-trifluoroacetylpiperidin-4-yl)-phenyl]ethyl]benzenesulfonamide.
$^1$H-NMR(DMSO-$d_6$) δ ppm:
1.48-1.65 (2H, m), 1.79-1.94 (2H, m), 2.58-3.04 (6H, m), 3.88-4.00 (1H, m), 4.35-4.47 (3H, m), 6.28 (1H, d, J=9.2Hz), 6.65-7.20 (5H, m), 7.50 (1H, dd, J=9.2Hz, 2.9Hz), 7.70-8.00 (3H, m), 8.30-8.65 (2H, br)

Example 2

The following compounds were prepared in a similar manner to that described in Example 1, optionally, by further purification by ODS column chromatography and/or by conversion to a desired hydrochloride in a usual way.

5-Amidino-N-[2-(4-isopropylphenyl)ethyl]-2-hydroxybenzene-sulfonamide hydrochloride (Compound 2)

$^1$H-NMR(DMSO-$d_6$) δ ppm:
1.16 (6H, d, J=6.9Hz), 2.60-2.71 (2H, m), 2.76-2.89 (1H, m), 2.94-3.06 (2H, m), 7.04 (2H, d, J=8.1Hz), 7.11 (2H, d,

J=8.1Hz), 7.19 (1H, d, J=8.6Hz), 7.38-7.50 (1H, m), 7.87 (1H, dd, J=8.6Hz, 2.4Hz), 8.14 (1H, d, J=2.4Hz), 8.82 (2H, s), 9.25 (2H, s), 12.03 (1H, s)

5-Amidino-N-[2-(4-isopropoxylphenyl)ethyl]-2-hydroxybenzene-sulfonamide hydrochloride (Compound 3)

$^1$H-NMR(DMSO-$d_6$) δ ppm:
1.22 (6H, d, J=6.0Hz), 2.50-2.70 (2H, m), 2.89-3.01 (2H, m), 4.45-4.60 (1H, m), 6.77 (2H, d, J=8.6Hz), 7.00 (2H, d, J=8.6Hz), 7.17 (1H, d, J=8.7Hz), 7.30-7.48 (1H, m), 7.86 (1H, dd, J=8.7Hz, 2.5Hz), 8.13 (1H, d, J=2.5Hz), 8.82 (2H, s), 9.24 (2H, s), 12.03 (1H, br-s)

5-Amidino-N-[2-(4-piperidin-4-yloxyphenyl)ethyl]-2-hydroxy-benzenesulfonamide dihydrochloride (Compound 4)

$^1$H-NMR(DMSO-$d_6$) δ ppm:
1.71-1.88 (2H, m), 1.98-2.13 (2H, m), 2.55-2.71 (2H, m), 2.89-3.32 (6H, m), 4.51-4.66 (1H, m), 6.87 (2H, d, J=8.6Hz), 7.06 (2H, d, J=8.6Hz), 7.23 (1H, d, J=8.7Hz), 7.40 (1H, t, J=5.5Hz), 7.88 (1H, dd, J=8.7Hz, 2.5Hz), 8.14 (1H, d, J=2.5Hz), 8.70-9.10 (4H, m), 9.29 (2H, s), 12.12 (1H, s)

(S)-5-Amidino-2-hydroxy-N-[2-(4-(3-pyrrolidinyloxy)phenyl)-ethyl]benzenesulfonamide dihydrochloride (Compound 5)

$^1$H-NMR (DMSO-$d_6$) δ ppm:
2.00-2.24 (2H, m), 2.57-2.61 (2H, m), 2.92-3.06 (2H, m), 3.15-3.37 (3H, m), 5.00-5.12 (1H, m), 6.85 (2H, d, J=8.7Hz), 7.08 (2H, d, J=8.7Hz), 7.26 (1H, d, J=8.7Hz), 7.35 (1H, t, J=5.8Hz), 7.90 (1H, dd, J=8.7Hz, 2.5Hz), 8.14 (1H, d, J=2.5Hz), 8.97 (2H, s), 9.20-9.58 (4H, m), 12.10 (1H, s)
Specific rotation: $[\alpha]_D^{29}$ = +4.0 ° (c=0.35, 1 mol/L hydrochloric acid)

3-Amidino-N-[2-(4-isopropylphenyl)ethyl]benzenesulfonamide hydrochloride (Compound 6)

$^1$H-NMR (DMSO-$d_6$) δ ppm:
1.17 (6H, d, J=6.9Hz), 2.60-2.71 (2H, m), 2.75-3.05 (3H, m), 7.06 (2H, d, J=8.1Hz), 7.13 (2H, d, J=8.1Hz), 7.77-8.14 (4H, m), 8.16-8.24 (1H, m), 9.10-9.70 (4H, m)

Example 3

5-Amidino-2-hydroxy-N-[2-[4-(4-piperidinyl)phenyl]ethyl]-benzenesulfonamide dihydrochloride (Compound 7)

5-Amidino-2-hydroxy-N-[2-[4-(1-trifluoroacetyl-piperidin-4-yl)phenyl]ethyl]benzenesulfonamide (1.52 g) was dissolved in 15 mL of methanol. To the solution was added a solution (8 mL) of 3.92 g of potassium carbonate in water, and the mixture was stirred at room temperature for 3 hours. The pH of the reaction mixture was adjusted to pH 1 with concentrated hydrochloric acid, and the mixture was concentrated under reduced pressure. Ethanol was added to the residue, and the mixture was concentrated under reduced pressure. The resulting residue was suspended in a mixture solution of ethanol-methanol (1:1), and the insoluble material was filtered off through Celite®. The filrate was concentrated under reduced pressure, the residue was suspended again in a mixture solution of ethanol-methanol (1:1), and the insoluble material was filtered off through Celite®. The solvent was removed under reduced pressure to give 1.49 g of 5-amidino-2-hydroxy-N-[2-[4-(4-piperidinyl)phenyl]ethyl]-benzenesulfonamide dihydrochloride.
$^1$H-NMR(DMSO-$d_6$) δ ppm:
1.69-1.96 (4H, m), 2.60-3.10 (7H, m), 7.10 (4H, s), 7.26 (1H, d, J=8.8Hz), 7.45 (1H, br-s), 7.80-8.00 (1H, m), 8.14 (1H, d, J=2.2Hz) 8.82-9.10 (4H, m), 9.31 (2H, s), 12.16 (1H, s)

Example 4

5-Amidino-2-hydroxy-N-[2-[4-[1-(1-iminoethyl)piperidin-4-yl]phenyl]ethyl]benzenesulfonamide dihydorchloride (Compound 8)

5-Amidino-2-hydroxy-N-[2- [4-(4-piperidinyl)phenyl]-ethyl]benzenesulfonamide dihydrochloride (1.46 g) and 761 mg of ethyl acetimidate hydrochloride were added to 20 mL of methanol, and 1.72 mL of triethylamine was added dropwise to the mixture. After being stirred at room temperature for 26 hours, the reaction mixture was concentrated under reduced pressure. The resulting residue was purified by ODS column chromatography (eluent: methanol-0.02 mol/L hydrochloric acid) to give 1.20 g of 5-amidino-2-hydroxy-N-[2-[4-[1-(1-iminoethyl)piperidin-4-yl]-phenyl]ethyl]

benzenesulfonamide dihydorchloride.
$^1$H-NMR(DMSO-$d_6$) δ ppm:
1.52-1.90 (4H, m), 2.30 (3H, s), 2.59-2.61 (2H, m), 2.76-2.91 (1H, m), 2.94-3.21 (3H, m), 3.90-4.05 (1H, m), 4.17-4.30 (1H, m), 7.08 (2H, d, J=8.2Hz), 7.14 (2H, d, J=8.2Hz), 7.28 (1H, d, J=8.7Hz), 7.43 (1H, t, J=5.7Hz), 7.91 (1H, dd, J=8.7Hz, 2.5Hz), 8.14 (1H, d, J=2.5Hz), 8.71 (1H, s), 9.03 (2H, s), 9.20-9.40 (3H, m), 12.18 (1H, s)

Example 5

The following compounds were prepared in a similar manner to that described in Example 4.

(S)-5-Amidino-2-hydroxy-N-[2-[4-[1-(1-iminoethyl)pyrrolidin-3-yloxylphenyl]ethyl]benzenesulfonamide dihydrochloride (Compound 9)

$^1$H-NMR(DMSO-$d_6$) δ ppm:
2.05-2.40 (5H, m), 2.55-2.72 (2H, m), 2.89-3.08 (2H, m), 3.25-4.05 (4H, m), 5.03-5.26 (1H, m), 6.76-6.93 (2H, m), 7.01-7.16 (2H, m), 7.24-7.50 (2H, m), 7.85-8.00 (1H, m), 8.09-8.12 (1H, m), 8.40-8.62 (1H, m), 9.06 (2H, s), 9.20-9.46 (3H, m), 12.23 (1H, br-s)
Specific rotation: $[\alpha]_D^{25}$ = +13.8° (c=0.55, 1 mol/L hydrochloric acid)

5-Amidino-2-hvdroxv-N-[2-[4-[1-(1-iminoethyl)piperidin-4-yloxy]phenyl]ethyl]benzenesulfonamide dihydrochloride (Compound 10)

$^1$H-NMR(DMSO-$d_6$) δ ppm:
1.64-1.81 (2H, m), 1.95-2.10 (2H, m), 2.30 (3H, s), 2.64 (2H, t, J=7.5Hz), 2.90-3.06 (2H, m), 3.43-3.90 (4H, m), 4.58-4.70 (1H, m), 6.88 (2H, d, J=8.6Hz), 7.06 (2H, d, J=8.6Hz), 7.30 (1H, d, J=8.8Hz), 7.38 (1H, t, J=5.9Hz), 7.92 (1H, dd, J=8.8Hz, 2.4Hz), 8.15 (1H, d, J=2.4Hz), 8.78 (1H, s), 9.07 (2H, s), 9.33 (4H, s), 12.20 (1H, s)

Test Example 1

Measurement of inhibitory activity for activated blood coagulation factor X

Five μL of dimethylsulfoxide solution of test compound, 375 μL of tris-hydrochloric acid buffer (pH 8.4) and 100 μL of 1 mM S-2222 (Daiichi Pure Chemicals) aqueous solution were mixed. Then 20 μL of 0.6 U/mL human activated blood coagulation factor X (Calbiochem Corporation) in gelatin-glycine buffer was added and the mixture was incubated for 10 minutes at 37 °C. The reaction was terminated with the addition of 100 μL of 60 % acetic acid and absorbance (405 nm) was measured.
The group without test compound was defined as control and the group without human activated blood coagulation factor X was defined as blank. The concentration of test compound that showed 50 % inhibition of control ($IC_{50}$) was obtained and this value was used as the index of inhibitory activity for activated blood coagulation factor X. Results are shown in Table 1.

Test Example 2

Measurement of inhibitory activity for thrombin

Five μL of dimethylsulfoxide solution of test compound, 375 μL of tris-hydrochloric acid buffer (pH 8.4) and 100 μL of 1 mM S-2238 (Daiichi Pure Chemicals) aqueous solution were mixed. Then 20 μL of 2.0 U/mL human thrombin (Sigma Chemical Company) in gelatin-glycine buffer was added and the mixture was incubated for 10 minutes at 37 °C. The reaction was terminated with the addition of 100 μL of 60 % acetic acid and absorbance (405 nm) was measured.
The group without test compound was defined as control and the group without human thrombin was defined as blank. The concentration of test compound that showed 50 % inhibition of control ($IC_{50}$) was obtained and this value was used as the index of inhibitory activity for thrombin. Results are shown in Table 1.

[Table 1]

| Compound | Inhibitory activity for activated blood coagulation factor X (nM) | Inhibitory activity for thrombin (μM) |
|---|---|---|
| 2 | 64 | 101 |
| 8 | 87 | >100 |
| 9 | 88 | >100 |
| 10 | 110 | >100 |

Test Example 3

Measurement of anticoagulation effects (plasma prothrombin time)

Two μL of dimethylsulfoxide solution of test compound which was put in the process tube was incubated at 37 °C, and 50 μL of normal human plasma (George King Bio-Medical Inc.) was mixed. One minute later, 100 μL of plasma prothrombin time reagent (Neoplastin Plus® , Boehringer Mannheim) was added into the mixture. Prothrombin time was measured with ST4 (Boehringer Mannheim).

The group without test compound was defined as control. The concentration of test compound that prolonged the clotting time of control by 2 times ($CT_2$) was obtained and this value was used as the index of anticoagulation activity. Results are shown in Table 2.

[Table 2]

| Compound | Anticoagulation effect (μM) |
|---|---|
| 8 | 0.21 |
| 9 | 0.18 |
| 10 | 0.22 |

Test Example 4

Acute toxicity test

Male Wistar rats aged 7 weeks (SLC) were divided into several groups consisting of 5 rats. Test compound was dissolved at the concentration that became the administration volume of

2.5 mL/kg. The solution was administered through tail vein at an infusion rate of 1 mL/minute. Observations were performed at constant intervals, and survival rate was judged for 24 hours. Results are shown in Table 3.

[Table 3]

| Compound | Administration dose (mg/kg) | Death cases |
|---|---|---|
| 8 | 30 | 0/5 |

**Claims**

**1.** A 3-amidinobenzenesulfonamide derivative represented by the general formula:

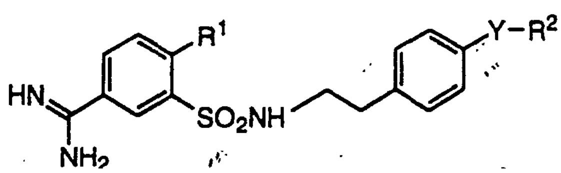

wherein $R^1$ represents a hydrogen atom or a hydroxy group; Y represents a single bond or an oxygen atom; and $R^2$ represents a lower alkyl group or a group represented by the general formula:

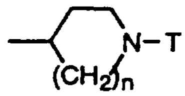

(wherein n represents 1 or 2; and T represents a hydrogen atom, a lower acyl group, a halo (lower acyl) group or a group represented by the general formula:

-C(=NH)-W

(wherein W represents a lower alkyl group)), or a pharmaceutically acceptable salt thereof.

**2.** A pharmaceutical composition comprising a 3-amidinobenzenesulfonamide derivative represented by the general formula:

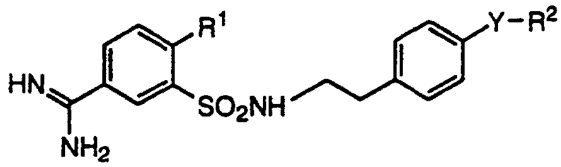

wherein $R^1$ represents a hydrogen atom or a hydroxy group; Y represents a single bond or an oxygen atom; and $R^2$ represents a lower alkyl group or a group represented by the general formula:

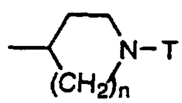

(wherein n represents 1 or 2; and T represents a hydrogen atom, a lower acyl group, a halo(lower acyl) group or a group represented by the general formula:

-C(=NH)-W

(wherein W represents a lower alkyl group)), or a pharmaceutically acceptable salt thereof.

**3.** An activated blood coagulation factor X inhibitor which comprises, as an active ingredient, a 3-amidinobenzenesulfonamide derivative represented by the general formula:

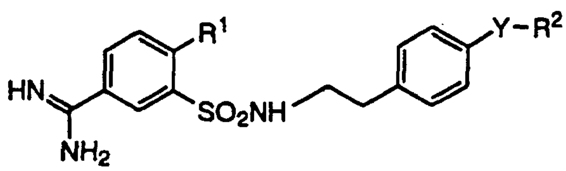

wherein $R^1$ represents a hydrogen atom or a hydroxy group; Y represents a single bond or an oxygen atom; and $R^2$ represents a lower alkyl group or a group represented by the general formula:

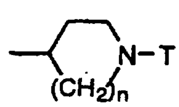

(wherein n represents 1 or 2; and T represents a hydrogen atom, a lower acyl group, a halo (lower acyl) group or a group represented by the general formula:

-C(=NH)-W

(wherein W represents a lower alkyl group)), or a pharmaceutically acceptable salt thereof.

**4.** A method for the prevention or treatment of thromboembolic diseases which comprises administering a 3-amidinobenzenesulfonamide derivative represented by the general formula:

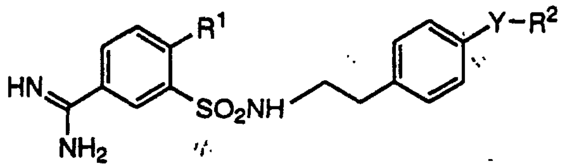

wherein $R^1$ represents a hydrogen atom or a hydroxy group; Y represents a single bond or an oxygen atom; and $R^2$ represents a lower alkyl group or a group represented by the general formula:

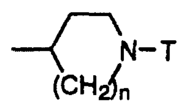

(wherein n represents 1 or 2; and T represents a hydrogen atom, a lower acyl group, a halo (lower acyl) group or a group represented by the general formula:

-C(=NH)-W

(wherein W represents a lower alkyl group)), or a pharmaceutically acceptable salt thereof.

**5.** A use of a 3-amidinobenzenesulfonamide derivative represented by the general formula:

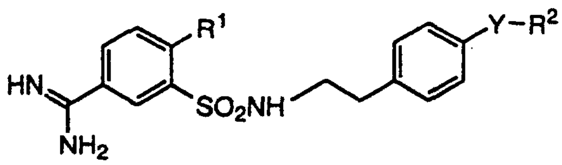

wherein $R^1$ represents a hydrogen atom or a hydroxy group; Y represents a single bond or an oxygen atom; and $R^2$ represents a lower alkyl group or a group represented by the general formula:

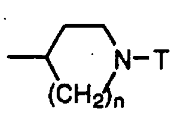

(wherein n represents 1 or 2; and T represents a hydrogen atom, a lower acyl group, a halo (lower acyl) group or a group represented by the general formula:

-C(=NH)-W

(wherein W represents a lower alkyl group)), or a pharmaceutically acceptable salt thereof for the manufacture of

a pharmaceutical composition for the prevention or treatment of thromboembolic diseases.

**6.** A process for the manufacture of a pharmaceutical composition for the prevention or treatment of thromboembolic diseases, **characterized in** the use, as an essential constituent of said pharmaceutical composition, of a 3-amidinobenzenesulfonamide derivative represented by the general formula:

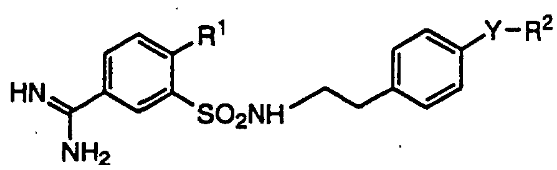

wherein $R^1$ represents a hydrogen atom or a hydroxy group; Y represents a single bond or an oxygen atom; and $R^2$ represents a lower alkyl group or a group represented by the general formula:

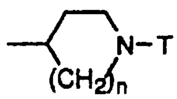

(wherein n represents 1 or 2; and T represents a hydrogen atom, a lower acyl group, a halo (lower acyl) group or a group represented by the general formula:

-C(=NH)-W

(wherein W represents a lower alkyl group)), or a pharmaceutically acceptable salt thereof.

**7.** A 3-cyanobenzenesulfonamide derivative represented by the general formula:

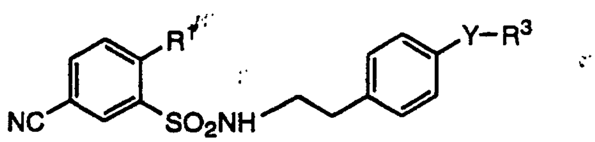

wherein $R^1$ represents a hydrogen atom or a hydroxy group; Y represents a single bond or an oxygen atom; and $R^3$ represents a lower alkyl group or a group represented by the general formula:

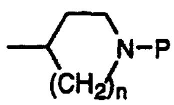

(wherein n represents 1 or 2; and P represents a hydrogen atom, a lower acyl group, a halo (lower acyl) group, a group represented by the general formula:

-C(=NH)-W

(wherein W represents a lower alkyl group) or an amino-protective group), or a salt thereof.

## INTERNATIONAL SEARCH REPORT

International application No.
PCT/JP00/01891

A. CLASSIFICATION OF SUBJECT MATTER
Int.Cl$^7$ C07C311/46, C07C311/19, C07C311/29, C07D207/12, C07D211/26, A61K31/40, A61P7/02, A61P43/00, A61K31/445, A61K31/18

According to International Patent Classification (IPC) or to both national classification and IPC

B. FIELDS SEARCHED

Minimum documentation searched (classification system followed by classification symbols)
Int.Cl$^7$ C07C311/46, C07C311/19, C07C311/29, C07D207/12, C07D211/26, A61K31/40, A61P7/02, A61P43/00, A61K31/445, A61K31/18

Documentation searched other than minimum documentation to the extent that such documents are included in the fields searched

Electronic data base consulted during the international search (name of data base and, where practicable, search terms used)
CAPLUS(STN), REGISTRY(STN)

C. DOCUMENTS CONSIDERED TO BE RELEVANT

| Category* | Citation of document, with indication, where appropriate, of the relevant passages | Relevant to claim No. |
|---|---|---|
| A | US, 5084466, A (Hoffmann-La Roche Inc.), 28 January, 1992 (28.01.92) & JP, 2-235853, A & EP, 381033, A1 | 1~7 |
| A | US, 4948809, A (Boehringer Mannheim GmbH), 14 August, 1990 (14.08.90) & JP, 62-84054, A & EP, 221344, A1 | 1~7 |

☐ Further documents are listed in the continuation of Box C. ☐ See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 18 July, 2000 (18.07.00) | 01 August, 2000 (01.08.00) |
| Name and mailing address of the ISA/ Japanese Patent Office | Authorized officer |
| Facsimile No. | Telephone No. |

Form PCT/ISA/210 (second sheet) (July 1992)